# EUROPÄISCHE PATENTSCHRIFT

(11) **EP 1 485 057 B1**
(45) Veröffentlichungstag und Bekanntmachung des Hinweises auf die Patenterteilung: **28.12.2005**
(21) Anmeldenummer: 03711903.9
(22) Anmeldetag: 27.02.2003
(51) Int. Cl.: A61K 6/00

(54) **DERIVATE VON AZA-SPIROVERBINDUNGEN ZUR BEHANDLUNG VON SCHMERZEN**
DERIVATIVES OF AZASPIRO COMPOUNDS FOR THE TREATMENT OF PAIN
DERIVES DE COMPOSES AZASPIRO POUR LE TRAITEMENT DE LA DOULEUR

(30) Priorität: 07.03.2002 DE 10210195
(43) Veröffentlichungstag der Anmeldung: 15.12.2004
(73) Patentinhaber: SCHWARZ PHARMA AG, 40789 Monheim/Rhld. (DE)
(72) Erfinder: MEESE, Claus, 40789 Monheim (DE); SELVE, Norma, 53842 Troisdorf (DE); SCHMIDT, Dirk, 40597 Düsseldorf (DE)
(86) Internationale Anmeldenummer: PCT/EP2003/001985
(87) Internationale Veröffentlichungsnummer: WO 2003/073979

(56) Entgegenhaltungen:
- GB-A- 995 864
- GB-A- 1 015 026
- US-A- 2 866 734
- US-A- 3 629 276
- OLDFIELD W ET AL: "THE CHEMISTRY AND PHARMACOLOGY OF A SERIES OF CYCLOALKANESPIRO-5'-HYDANTOINS" JOURNAL OF MEDICINAL CHEMISTRY, AMERICAN CHEMICAL SOCIETY. WASHINGTON, US, Bd. 8, 1965, Seiten 239-249, XP000615331 ISSN: 0022-2623
- LESPAGNOL A ET AL: "Etude de quelques dérivés de l'oxazolidinedione" ANNALES PHARMACEUTIQUES FRANCAISES , Bd. 10, 1951, Seiten 15-36, XP009017331
- ULBRICHT, H.: "2-Aminooxazoles as potential hydrogen-bonding virucides. Part 1. N-Unsubstituted and N-substituted 2-aminooxazoles" PHARMAZIE , Bd. 42, Nr. 9, 1987, Seiten 598-601, XP001154960
- HARNDEN ET AL: "Synthesis of compounds with potential central nervous system stimulant activity. II. 5-Spiro-substituted 2-amino-2-oxazolines" JOURNAL OF MEDICINAL CHEMISTRY, AMERICAN CHEMICAL SOCIETY. WASHINGTON, US, Bd. 13, Nr. 2, 1970, Seiten 305-308, XP002082528 ISSN: 0022-2623
- NEWMAN M ET AL: "New Reactions Involving Alkaline Treatment of 3-Nitroso-2-Oxazolidones" JOURNAL OF THE AMERICAN CHEMICAL SOCIETY, Bd. 73, 1951, Seiten 4199-4204, XP002254807
- DATABASE CROSSFIRE BEILSTEIN [Online] Beilstein Institut zur Förderung der Chemischen Wissenschaften, Frankfurt am Main, DE; Database accession no. BRN 125422 XP002254808 & GRANGER ET AL: TRAV. SOC.PHARM.MONTPELLIER, Bd. 24, 1964, Seite 244
- REGNIER ET AL: "Synthèse et propriétés pharmacologiques des dérivés des oxa-1 oxo-2 aza-3 spirannes" CHIMIE TH RAPEUTIQUE, Bd. 3, 1969 - 1969, Seiten 174-184, XP009017336
- ROBBE Y ET AL: "RADIOPROTECTION CHIMIQUE COMPAREE DE DIVERSES STRUCTURES HETEROCYCLIQUES PENTOGONALES A DEUX HETEROATOMES" EUROPEAN JOURNAL OF MEDICINAL CHEMISTRY, EDITIONS SCIENTIFIQUE ELSEVIER, PARIS, FR, Bd. 17, Nr. 3, 1982, Seiten 235-243, XP001015842 ISSN: 0223-5234

## Beschreibung

Neuropathische Schmerzen sind eine schwierig zu behandelnde Form von chronischen Schmerzen, die durch Verletzungen oder Erkrankungen des peripheren und/oder zentralen Nervensystems hervorgerufen werden und die nur schlecht auf traditionelle Analgetika ansprechen.

Auf Grund der Ähnlichkeiten in der Pathophysiologie von Epilepsie und neuropathischem Schmerz werden in jüngster Zeit zunehmend antikonvulsive Wirkstoffe zur Behandlung von neuropathischem Schmerz herangezogen. Ein Beispiel hierfür ist Gabapentin, dass als Antiepileptikum bereits längere Zeit zugelassen ist, in jüngster Zeit aber auch zunehmend Bedeutung in der Behandlung von neuropathischem Schmerz bekommt (Tremont-Lukats, Drugs 60, 2000, 1029; Block, Nervenarzt 72, 2001, 69).

Obwohl der Wirkmechanismus von Gabapentin noch nicht völlig aufgeklärt ist, hat Gabapentin durch seine Beeinflussung der glutaminergen/GABAergen Übertragung und der Beeinflussung von Calciumkanälen ein breites Wirkspektrum, das von der Behandlung der Epilepsie über neuropathische und andere Schmerzzustände, wie z.B. Migräneschmerz (Block, Nervenarzt 72, 2001, 69) oder Muskel- und Skelettschmerz (EP 1 047 414), bis hin zur Behandlung von Depressionen (EP 552 240), neurodegenerativen Erkrankungen (EP 446 570), Angst- und Panikzuständen (EP 804182) oder Manien (EP 825 857) reicht.

Ein Nachteil von Gabapentin ist die Bildung von toxischem Gabapentin-Lactam (2-Azaspiro[4.5]decan-3-on) bei Lagerung, und die damit verbundene Schwierigkeit, stabile Gabapentin-Formulierungen herzustellen.

In der WO 99/25683 werden eine große Zahl von in Position 4 substituierten Pyrrolidinon-Verbindungen, die auch Azaspiroverbindungen wie Gabapentin-Lactam umfassen, zur Behandlung von Erkrankungen vorgeschlagen, die mit erhöhten Glutamatspiegeln einhergehen, wie z.B. Epilepsie, Alzheimer, ALS oder Parkinson. In einem in-vitro Modell wird die Wirksamkeit des Gabapentin-Lactams in Ischämien und die Reduktion des Glutamat-Spiegels gezeigt. Ferner wird die neuroprotektive Wirkung von Gabapentin-Lactam in einem Ratten-Modell demonstriert. Auf Grund der Toxizität von Gabapentin-Lactam ist dies jedoch nicht zur Therapie am Menschen geeignet. Zudem gibt es in der WO 99/25683 keinen Hinweis darauf, dass die beanspruchten Pyrrolidone zur Behandlung von neuropathischem Schmerz geeignet sind.

In der DE 25 57 220 werden N-substituierte Gabapentin-Lactam-Derivate zur Behandlung von Epilepsie und zerebralen Störungen beschrieben. Die Verwendung bei Schmerzen wird nicht gelehrt.

Azaspiroverbindungen mit Aryl-Substituenten zur Behandlung von Schmerzen werden beschrieben in EP 337 547, EP 687 268, EP 880 528, EP 894 497, EP 906 315, EP 912 579, EP 929 554, EP 977 758 und EP 989 987. Es findet sich in diesen Dokumenten kein Hinweis darauf, dass auch Desaryl-Azaspiroverbindungen analgetisches Potential haben.

EP A 116 347 schlägt Amino-substituierte 1-Azaspiro[4.5.]decane und -undecane zur Behandlung von Schmerzen vor. 2-Azaspiroverbindurigen oder 1,3-Diazaspiroverbindungen werden nicht offenbart.

OLDFIELD, J.MED. CHEM., 8, 1965, 239 beschreibt cycloalkanspiro-5'-hydantoine zur Behandlung von Schmuz.

In der klinischen Praxis haben sich nur wenige Wirkstoffe bei der Behandlung von chronischen oder neuropathischen Schmerzen als wirksam und geeignet erwiesen, so dass in dieser Indikation weiterhin ein grosser Bedarf an innovativen Arzneimitteln besteht

Ziel der vorliegenden Erfindung war es daher, alternative Arzneimittel zur Behandlung von Schmerz, insbesondere von chronischem, chronisch-entzündlichem und/oder neuropathischem Schmerz, zur Verfügung zu stellen.

Dabei wurde überraschenderweise gefunden, dass von Gabapentin-Lactam abgeleitete Azaspiroverbindungen wie beansprucht eine stärkere analgetische Potenz als Gabapentin und Gabapentin-Lactam aufweisen und zugleich eine geringere Toxizität als Gabapentin-Lactam zeigen.

Die erfindungsgemäßen Azaspiroverbindungen, die zur therapeutischen Verwendung geeignet sind, lauten wie folgt
1-Oxa-3-Aza-Spiro(4,5)decan-2-on,
1-Oxa-3-Aza-Spiro(4,5)decan-2-thion,
1-3-Diaza-Spiro(4,5)decan-2-on
1-3-Diaza-Spiro(4,5)decan-2-thion
1-3-Diaza-Spiro(4,5)decan-2,4-di-thion
1-3-Diaza-Spiro(4,5)decan-2,4-dion

Gegenstand der vorliegenden Erfindung ist daher eine pharmazeutische Zusammensetzung zur oralen Verabreichung umfassend 1,3-Diazaspiro[4,5]decan-2,4-dithion oder dessen Pharmazeutisch annehmbare Salze sowie mindestens einen pharmazeutisch annehmbaren Hilfsstoff, sowie die

Verwendung von 1,3-Diazaspiro[4,5]decan-2,4-dithion oder eines möglichen Tautomers und/oder pharmazeutisch annehmbaren Salzes zur Herstellung eines Arzneimittels zur Behandlung von Schmerzen, sowie die
Verwendung einer Substanz, ausgewählt aus
1-Oxa-3-Aza-Spiro(4,5)decan-2-on,
1-Oxa-3-Aza-Spiro(4,5)decan-2-thion,
1-3-Diaza-Spiro(4,5)decan-2-on
1-3-Diaza-Spiro(4,5)decan-2-thion
1-3-Diaza-Spiro(4,5)decan-2,4-di-thion
1-3-Diaza-Spiro(4,5)decan-2,4-dion
sowie von deren pharmazeutisch akzeptablen Salzen zur Herstellung eines Arzneimittels zur Behandlung von chronischem und/oder neuropathischem Schmerz.

Die Azaspiroverbindungen können als freie Base oder als pharmazeutisch annehmbare Salze vorliegen und sind in beiden Formen Gegenstand der Erfindung.

Weiterhin können die Azaspiroverbindungen in Abhängigkeit von Substituenten in verschiedenen tautomeren Formen vorliegen, die gegebenenfalls durch Salzbildung stabilisiert werden können. Auch diese Tautomeren und ihre Salze sind Gegenstand der Erfindung.

Als pharmazeutisch annehmbare Salze kommen alle bioverträglichen Salze in Frage, die die pharmakologischen Eigenschaften der Wirkstoffe weitgehend erhalten und keine unerwünschten toxischen Effekte auslösen. Beispiele hierfür sind insbesondere Additionssalze anorganischer oder organischer Säuren, wie z.B. Hydrogenchlorid, Hydrogenbromid, Essigsäure, Zitronensäure, Weinsäure, Oxalsäure, Fumarsäure, Äpfelsäure, Bernsteinsäure oder Methansulfonsäure.

Ferner ist dem Fachmann klar, dass in Abhängigkeit von den Substituenten Azaspiroverbindungen in optisch inaktiver oder aktiver Form existieren können. Daher werden reine Enantiomere ebenso wie Razemate oder optisch inaktive Verbindungen explizit zum Gegenstand der Erfindung gemacht.

Ein weiterer Gegenstand der Erfindung sind pharmazeutische Zusammensetzungen, die eine Azaspiroverbindung der Formeln wie oben beschrieben, sowie mindestens einen pharmazeutisch annehmbaren Hilfsstoff umfassen.

Dem Fachmann ist klar, dass die pharmazeutische Formulierung in Abhängigkeit vom beabsichtigten Applikationsweg unterschiedlich ausgestaltet sein kann. So kann die pharmazeutische Formulierung beispielsweise zur oralen, Verabreichung angepasst sein.

Entsprechende Formulierungen und hierfür geeignete pharmazeutische Träger bzw. Hilfsstoffe wie Füllstoffe, Sprengmittel, Bindemittel, Gleitmittel, Stabilisatoren, Aromastoffe, Antioxidanzien, Konservierungsmittel. Dispersions- oder Lösungsmittel, Puffer oder Elektrolyte, sind dem Fachmann auf dem Gebiet der Pharmazeutik bekannt und sind beispielsweise in Standardwerken wie Sucker, Fuchs und Speiser ("Pharmazeutische Technologie", Georg Thieme Verlag, Stuttgart) beschrieben.

Die Verbindungen der Erfindung und die diese enthaltenden pharmazeutischen Zusammensetzungen werden oral verabreicht und können beispielsweise als Kapsel, Tablette, Pulver, Granulat, Dragee oder in flüssiger Form vorliegen.

Dabei kann die Formulierung als schnell freisetzende Darreichungsform ausgestaltet sein, wenn ein rascher Wirkeintritt gewünscht ist, z.B. bei akutem, bzw. akut durchbrechendem chronischen bzw. neuropathischen Schmerz. Entsprechende orale Formulierungen sind beispielsweise beschrieben in EP 159 237 oder EP 1 126 821.

Ist dagegen eine protrahierte Freisetzung erwünscht, bietet sich eine Formulierung mit retardierter Wirkstofffreisetzung an. Entsprechende orale Formulierungen sind ebenfalls aus dem Stand der Technik bekannt.

Ein weiterer Gegenstand der Erfindung sind Verkaufspackungen, umfassend mindestens eine pharmazeutische Formulierung, wie oben beschrieben, sowie eine Anleitung zu deren Verwendung. Eine solche Verkaufspackung kann auch weitere Arzneimittel enthalten. Zum Beispiel könnte die Verkaufspackung zusätzlich ein weiteres Analgetikum, ein Sedatiwm, ein Ergotamin-Derivat, ein Antiemetikum, ein Antiphlogistikum oder ein Antidepressivum enthalten.

Überraschenderweise zeigen die erfindungsgemäßen Verbindungen in pharmakologischen Vergleichstests eine starke Wirksamkeit im Formalintest, einem in-vivo Test für die Voraussage der potentiellen Wirksamkeit einer Substanz bei der Behandlung von chronischem, bzw. chronisch-entzündlichem und/oder neuropathischem Schmerz (Tjolsen and Herle, Handbook Exp. Pharmacol. Vol 130, Ed: Dickenson & Besson, Springer Verlag 1997, Seite 6).

Abbildung 1 und Tabelle 1 zeigen die Reaktionen von Versuchstieren 20-45 Minuten nach intraperitonealer Gabe ausgewählter Verbindungen. Dabei wurden jeweils als maximale Dosierung Konzentrationen der Azaspiroverbindungen gewählt, die um ca. einen Faktor 2 unterhalb der toxischen Dosis liegen, die zuvor im IRWIN-Test (Irwin, Psychopharmacologia 13 (1968) 222) bestimmt wurde.

Dabei haben die in Abbildung 1 verwendeten Abkürzungen die folgende Bedeutung: SPM 10011 entspricht 1,3-Diazaspiro[4.5]decan-2,4-dion. SPM 10013 entspricht 1,3-Diazaspiro[4.5]decan-2,4-dithion. SPM 10019 entspricht 1,3-Diazaspiro[4.5]decan-2-on. GBP bedeutet Gabapentin. In der Legende wird jeweils in Klammern hinter der Substanzbezeichnung die Dosierung der Substanzen in mg/kg Körpergewicht angegeben.

Wie aus Abbildung 1 und aus Tabelle 1 hervorgeht, haben die erfindungsgemäßen Verbindungen überraschenderweise im Formalintest eine signifikant höhere Potenz als Gabapentin und Gabapentin-Lactam, die als Vergleich eingesetzt wurden.

Zudem erwiesen sich die oberhalb der maximal tolerablen Dosen einsetzenden Nebenwirkungen der erfindungsgemäßen Verbindungen (Sedierung, Tremor, Hypothermie) als weit weniger schwer als diejenigen des GPL, bei dem eine signifikante Letalitätsrate zu beobachten war.

**Tabelle 1**

| Verbindung | Dosierung (mg/kg) | mittlere Abweichung der Schmerzreaktion gegenüber Kontrolle (%) (x Minute nach Formalingabe) | | |
|---|---|---|---|---|
| | | 20-25' | 30-35' | 40-45' |
| SPM10011 | | | | |
| Meßreihe 1 | 64 | - 100 | -100 | - 80 |
| (n=10) | 32 | - 82 | - 64 | - 64 |
| | 16 | - 75 | - 11 | - 55 |
| | | | | |
| Meßreihe 2 | 64 | - 100 | - 100 | - 97 |
| (n=10) | 16 | - 84 | - 21 | - 34 |
| | 4 - | - 62 | (+) | ± 0 |

| SPM 10013 | | | | |
|---|---|---|---|---|
| Meßreihe 1 | 64 | - 98 | - 95 | - 78 |
| (n=10) | 32 | - 82 | - 60 | - 30 |
| | 16 | - 60 | - 14 | - 49 |
| | | | | |
| SPM 10019 | 32 | - 52 | ± 0 | - 40 |
| (n=10) | | | | |

| GPL | | | | |
|---|---|---|---|---|
| Meßreihe 2 | 32 | - 64 | - 15 | - 46 |
| (n=10) | 16 | - 74 | -40 | - 41 |
| | 8 | - 58 | -18 | - 15 |
| | | | | |
| Meßreihe 1 | 32 | (+) | -10 | n.b. |
| (n=10) | 16 | (+) | (+) | n.b. |
| | 8 | (+) | (+) | n.b. |
| | | | | |
| Morphium* | 8 | - 87 | -95 | - 88 |
| (+): Verstärkung der Schmerzreaktion n.b. = nicht bestimmt | | | | |

| | | | | |
|---|---|---|---|---|
| *) Mittelwert aus 9 Meßreihen | | | | |

Die erfindungsgemäß für die Therapie geeigneten Azaspiroverbindungen sind somit besonders zur Behandlung von Schmerzen, insbesondere von chronischen, chronisch-entzündlichen und/oder neuropathischen Schmerzen geeignet.

Ein Gegenstand der Erfindung ist daher die Verwendung einer Azaspiroverbindung der Formeln
1-Oxa-3-Aza-Spiro(4,5)decan-2-on,
1-Oxa-3-Aza-Spiro(4,5)decan-2-thion,
1-3-Diaza-Spiro(4,5)decan-2-on
1-3-Diaza-Spiro(4,5)decan-2-thion
1-3-Diaza-Spiro(4,5)decan-2,4-di-thion
1-3-Diaza-Spiro(4,5)decan-2,4-dion
sowie möglicher Tautomere und/oder pharmazeutisch annehmbarer Salze
zur Herstellung eines Arzneimittels zur Behandlung von chronischen, chronisch-entzündlichen und/oder neuropathischen Schmerzen.

Zur Herstellung eines Schmerzmittels besonders bevorzugt werden solche Azaspiroverbindungen, die im Formalintest, wie in Beispiel 7 beschrieben, in mindestens einem, bevorzugt in mindestens zwei der Testzeiträume (20-25', 30-35', 40-45' nach Formalingabe) eine mittlere Abweichung der Schmerzreaktion von wenigstens - 40 %, bevorzugt mindestens - 50 % oder - 60 % bewirken.

Unter dem Begriff "mittlere Abweichung der Schmerzreaktion" wird in dieser Anmeldung die relative Abweichung verstanden, die sich ergibt, wenn die gemittelte Zeit der Schmerzreaktion von 10 mit Wirkstoffen behandelten Tieren einer Versuchsreihe, wie in Ausführungsbeispiel 7 beschrieben, in einem definierten Zeitraum (20-25', 30-35' oder 40-45' nach Formalininjektion) mit der gemittelten Zeit der Schmerzreaktion von 10 mit Vehikel behandelten Kontrolltieren verglichen wird. Eine Reduktion der Schmerzreaktion wird dabei in negativen Prozentzahlen angegeben.

Die die erfindungsgemäßen Azaspiroverbindungen enthaltenden Arzneimittel können grundsätzlich zur Behandlung verschiedener Schmerzformen, wie z.B. Migräneschmerzen, Skelett- und Muskeischmerzen etc. verwendet werden. Besonders geeignet sind die die erfindungsgemäßen Azaspiroverbindungen enthaltenden Arzneimittel aber zur Behandlung von chronischen, chronisch-entzündlichen und/oder neuropathischen Schmerzen.

Neuropathischer Schmerz ist eine komplexe Erkrankung, die oft als Folgeerkrankung von Verletzungen, Infektionen, metabolischen Störungen und degenerativen Erkrankungen des Nervensystems auftreten kann. Beispiele für neuropathische Schmerzsyndrome sind Phantomschmerzen, postherpetische Neuralgien nach Gürtelrose, schmerzhafte diabetische Neuropathien, komplexe regionale Schmerzsyndrome, verschiedene Arten von Krebsschmerzen, neuropathische Schmerzen in Zusammenhang mit Multipler Sklerose oder mit Verletzungen größerer Nervenbahnen, dem Rückenmark oder dem Stammhirn.

Wie aus Abbildung 1 hervorgeht, sind insbesondere die erfindungsgemäßen Azaspiroverbindungen zur Behandlung von chronischem, chronisch-entzündlichem und/oder neuropathischem Schmerz geeignet.

### 1. Herstellung,von 1-Oxa-3-aza-spiro[4.5]decan-2-on

0,65 g (5,0 mmol)1-(Aminomethyl)-cyclohexanol, 1,2 g (5,5 mmol) Di-tert.-butyldicarbonat ((Boc)₂O), 0,611 g 4-Dimethylaminopyridin wurden in 50 ml Acetonitril gelöst und über Nacht bei Raumtemperatur gerührt. Anschließend wurde vollständig einrotiert, der Rückstand in 20 ml Essigsäure gelöst und wieder einrotiert. Der Rückstand wurde in 25 ml 1 M Salzsäure aufgenommen und mit 50 ml Toluol extrahiert.

Nach Abziehen des Toluols und Umkristallisieren aus Metyl-tert.-butylether fiel das Produkt als farblose, nadelförmige Kristalle aus.

Die Ausbeute betrug 15,5 % d.Th.
NMR (CDCl₃): 159.97; 82.82; 51.32; 36.36; 24.64; 22.23

### 2. Herstellung von 1-Oxa-3-aza-spiro[4.5]decan-2-thion

4,3 g (33,3 mmol) 1-(Aminomethyl)-cyclohexanol und 8,16 g (41,2 mmol) N,N'-Thiocarbonyldiimidazol (techn. 90 %ig) wurden in 250 ml Dichlormethan gelöst und 1 Stunde bei Raumtemperatur stehen gelassen. Der Ansatz wurde mit 250 ml 1 M Salzsäure extrahiert und die organische Phase vollständig einrotiert.

Der Gesamtrückstand wurde aus 40 ml Ethylacetat umkristallisiert. Das Produkt kristallisierte bei Raumtemperatur. Zur Vervollständigung der Kristallisation wurde der Ansatz über Nacht bei -25°C stehen gelassen. Der Überstand wurde abgesaugt, die Kristalle mit 5 ml Methyl-tert.-butylether gewaschen und im Vakuumtrockenschrank bei Raumtemperatur getrocknet.

Die Ausbeute betrug 4.4 g (77.2 % d. Theorie)

Der Schmelzpunkt wurde mit 152,0°C bestimmt.
NMR (CDCl₃): 188.35; 90.64; 54.07; 35.50; 24.35; 22.29.

### 3. Herstellung von 1,3-Diaza-spiro[4.5]decan-2,4-dion

Eine Lösung von 4,9 g (5,15 ml, 50 mmol) Cyclohexanon, 4,88 g (75 mmol) Kaliumcyanid und 14,4 g (150 mmol) Ammoniumcarbonat in 100 ml wäßrigem 50 %igem Ethanol wurde im Ölbad bei 65°C für 24 Stunden gerührt. Die Mischung wurde dann mit 300 ml Wasser verdünnt und 15 Min. refluxiert. Anschließend wurde auf 0°C abgekühlt und die Mischung auf 300 ml kalte 6N Salzsäure gegossen. Der Niederschlag wurde abfiltriert, mit kaltem Wasser gewaschen und im Vakuumtrockenschrank getrocknet.

Die Ausbeute betrug 7,23g (86 %).

In Dünnschichtchromatographie (Kieselgel 60, n-Heptan/Ethylacetat 1/1; Entwicklung mit verdünnter KMnO₄-Lösung) ergab sich ein einheitliches Produkt mit einem R_{f}-Wert von 0.24.

Der Schmelzpunkt wurde mit 218,2°C bestimmt.
NMR (CD₃OD): 180.92; 158.87; 64.38; 34.58; 25.80; 22.45.

### 4. Herstellung von 1,3-Diaza-spiro[4.5]decan-2,4-dithion

Eine Lösung von 1 g (6,0 mmol)1,3-Diaza-spiro[4.5]decan-2,4-dion (siehe Beispiel 3; schwer löslich) in 50 ml Toluol wurde unter Stickstoff mit 2,47 g Lawesson-Reagenz (2,4-Bis-(4-methoxyphenyl)-2,4-dithloxo-1,3,2,4-dithiadiphosphetan) versetzt und unter Stickstoff (115°C) für 26 h refluxiert. Die Lösung wurde auf Raumtemperatur abgekühlt und über Glaswolle filtriert. Das Filtrat wurde eingeengt und über Flash-Chromatographie gereinigt (Heptan / Ethylacetat 9:1).

Die Ausbeute betrug 781 mg (65%).

In Dünnschichtchromatographie (Kieselgel 60, n-Heptan/Ethylacetat 1/1; Entwicklung mit alkalischer KMn04-Lösung) ergab sich ein einheitliches Produkt mit einem Rf-Wert von 0,94 (Edukt: 0,31).

Der Schmelzpunkt wurde mit 271°C bestimmt.
NMR (DMSO-d6): 212.19, 180.45, 77.41, 36.97, 24.68, 21.15.

### 5. Herstellung von 1,3-Diaza-spiro[4.5]decan-2-on

Zu 1,6 g (9.5 mmol) 1,3-Diaza-spiro[4.5]decan-2,4-dion (siehe Beispiel 3) in 20 ml Diethylether wurden 20 ml einer 1 M Lösung von Lithiumaluminiumhydrid in Diethylether bei 0°C gegeben. Die Mischung wurde für 4 Stunden bei Raumtemperatur gerührt. Anschließend wurde die Mischung auf 0°C gekühlt und mit 3 ml H₂O, 2 ml 15 %iger Natronlauge und 5 ml H₂O versetzt. Die Lösung wurde mit 80 ml heißem Ethanol versetzt und filtriert. Das Filtrat wurde einrotiert, in Ethylacetat aufgenommen und erneut filtriert. Einengen des Filtrates lieferte 1,2 g farblos kristallines Produkt in 82% Ausbeute.

In Dünnschichtchromatographie (Kieselgel 60, n-Heptan/Ethylacetat 1/1; Entwicklung mit alkalischer KMnO₄-Lösung) ergab sich ein einheitliches Produkt mit einem R_{f}-Wert von 0.03.

Der Schmelzpunkt wurde mit 221°C bestimmt.
NMR (CDCl₃): 163.12; 57.67; 51.96; 37.58; 25.03; 22.65.

### 6. Herstellung von 1,3-Diaza-spiro[4.5]decan-2-thion

Diese Verbindung kann hergestellt werden, indem 1,3-Diaza-spiro[4.5]decan-2-on wie in Beispiel 5 beschrieben hergestellt wird und sodann wie in Beispiel 4 beschrieben mit Lawesson Reagenz umgesetzt wird.

### 7. In vivo-Test zur Bestimmung der analgetischen Wirksamkeit der Azaspiroverbindungen

Der Test wurde durchgeführt wie durch Wheeler-Aceto (Psychopharmacology 104, 1991, 35) beschrieben.

NMRI-Mäuse mit einem Gewicht von 20-25 g wurden unter kontrollierten Bedingungen (22 ± 2°C, 40-70 % Luftfeuchtigkeit) gehalten. 25 µl einer 5 % Formcarbonyllösung wurde in die Hinterpfote injiziert und nachfolgend zu definierten Zeitpunkten (20, 30, 40 Minuten) für jeweils 5 Minuten die Zeit des Leckens der Pfoten gemessen.

Die höchstmögliche einsetzbare, nicht-toxische Konzentration der jeweiligen Testsubstanzen wurde zunächst im IRWIN-Test bestimmt (Irwin, Psychopharmacologia 13,1968, 222).

Die Testsubstanzen wurden dann für den Formalin-Test in physiologischer Kochsalzlösung mit 0,5 % Natriumcarboxymethylzellulose gelöst und jeweils in 1-3 Dosierungen gemessen, die 10 Minuten vor Formalingabe intraperitoneal appliziert wurden. Als Vergleichswert diente eine Vehikelkontrolle (10 ml/kg).

Der Test wurde verblindet mit je 10 Mäusen pro Versuchsreihe durchgeführt. Die Auswertung erfolgte durch einen Vergleich der behandelten Tiere mit Vehikelkontrollen zu drei verschiedenen Zeitpunkten. Hierzu wurde jeweils für die 10 Tiere einer Versuchsreihe pro Zeitraum ein Mittelwert für die Schmerzreaküon ermittelt und dann die relative Abweichung der mit Wirkstoff behandelten Tiere von den Kontrolltieren für jeden der drei verschiedenen Zeiträume bestimmt. Eine mittlere Reduktion der Schmerzreaktion für die behandelten Tiere um 50 % ergibt sich demzufolge, wenn für einen definierten Zeitraum (z.B. 30-35' nach Formalininjektion) die Zeit des Leckens der Pfote (gemittelt über die 10 Versuchstiere) gegenüber unbehandelten Tieren um 50 % reduziert ist. Die statistische Signifikanz wurde mittels Mann-Whitney U-Test ermittelt.

## Patentansprüche

1. Pharmazeutische Zusammensetzung zur oralen Verabreichung umfassend 1,3-Diazaspiro[4,5]decan-2,4-dithion oder dessen pharmazeutisch annehmbare Salze sowie mindestens einen pharmazeutisch annehmbaren Hilfsstoff.

2. Verwendung von 1,3-Diazaspiro[4,5]decan-2,4-dithion oder eines möglichen Tautomers und/oder pharmazeutisch annehmbaren Salzes zur Herstellung eines Arzneimittels zur Behandlung von Schmerzen.

3. Verwendung einer Substanz, ausgewählt aus
1-Oxa-3-Aza-Spiro(4,5)decan-2-on,
1-Oxa-3-Aza-Spiro(4,5)decan-2-thion,
1-3-Diaza-Spiro(4,5)decan-2-on
1-3-Diaza-Spiro(4,5)decan-2-thion
1-3-Diaza-Spiro(4,5)decan-2,4-di-thion
1-3-Diaza-Spiro(4,5)decan-2,4-dion
sowie von deren pharmazeutisch akzeptablen Salzen zur Herstellung eines Arzneimittels zur Behandlung von chronischem und/oder neuropathischem Schmerz.

## Claims

1. Pharmaceutical composition for oral administration comprising 1,3-diazaspiro[4.5]decane-2,4-dithione or pharmaceutical acceptable salts thereof as well as at least one pharmaceutically acceptable adjuvant.

2. Use of 1,3-diazaspiro[4.5]decane-2,4-dithione or a possible tautomer and/or a pharmaceutically acceptable salt for producing a medicament for the treatment of pain.

3. Use of a substance, selected from among
1-oxa-3-aza-spiro[4.5]decane-2-one
1-xoa-3-aza-sprio[4.5]decane-2-thione
1-3-diaza-spiro[4.5]decane-2-one
1-3-diaza-spiro[4.5]decane-2-thione
1-3-diaza-spiro[4.5]decane-2,4-dithione
1-3-diaza-spiro[4.5]decane-2,4-dione
as well as the pharmaceutically acceptable salts thereof for producing a medicament for the treatment of chronic and/or neuropathic pain.

## Revendications

1. Composition pharmaceutique pour administration par voie orale, contenant de la 1,3-diazaspiro[4,5]décane-2,4-dithione ou des sels pharmaceutiquement acceptables de celle-ci ainsi qu'au moins un adjuvant pharmaceutiquement acceptable.

2. Utilisation de la 1,3-diazaspiro[4,5]décane-2,4-dithione ou d'un tautomère possible et/ou d'un sel pharmaceutiquement acceptable pour la fabrication d'un médicament destiné au traitement des douleurs.

3. Utilisation d'une substance sélectionnée parmi :
la 1-oxa-3-azaspiro[4,5]décane-2-one,
la 1-oxa-3-azaspiro[4,5]décane-2-thione,
la 1-3-diazaspiro[4,5]décane-2-one,
la 1-3-diazaspiro[4,5]décane-2-thione,
la 1-3-diazaspiro[4,5]décane-2,4-dithione,
la 1-3-diazaspiro[4,5]décane-2,4-dione,
ainsi que de sels pharmaceutiquement acceptables de celles-ci pour la fabrication d'un médicament destiné au traitement des douleurs chroniques et/ou neuropathiques.
